# EUROPÄISCHE PATENTSCHRIFT

(11) **EP 0 534 445 B1**
(45) Veröffentlichungstag und Bekanntmachung des Hinweises auf die Patenterteilung: **24.03.1999**
(21) Anmeldenummer: 92116415.8
(22) Anmeldetag: 24.09.1992
(51) Int. Cl.: A61K 31/685

(54) **Verfahren zur Herstellung eines Arzneimittels zur oralen oder topischen Verabreichung bei der Behandlung von Leishmaniasis**
Process for the preparation of a medicament for oral or topical administration in the treatment of leishmaniasis
Procédé de préparation d'un médicament pour l'administration orale ou topique dans le traitement de leishmaniasis

(30) Priorität: 27.09.1991 DE 4132344
(43) Veröffentlichungstag der Anmeldung: 31.03.1993
(73) Patentinhaber: Max-Planck-Gesellschaft zur Förderung der Wissenschaften e.V., D-37073 Göttingen (DE); ASTA Medica Aktiengesellschaft, D-01277 Dresden (DE)
(72) Erfinder: Eibl, Hansjörg, Prof. Dr., W-3406 Bovenden-Eddigehausen (DE); Unger, Clemens, Dr., W-3400 Göttingen (DE); Engel, Jürgen, Prof. Dr., W-8755 Alzenau (DE)
(74) Vertreter: Huber, Bernhard, Dipl.-Chem.

(56) Entgegenhaltungen:
- EP-A- 0 108 565
- EP-A- 0 230 575
- BIOCHEMICAL PHARMACOLOGY, Band 36, Nr. 16, 1987, Seiten 2633-2636, Pergamon Journals Ltd, GB; S.L. CROFT et al.: "The activity of alkyl phosphorylcholines and related derivatives against leishmania donovani"
- ANTIMICROBIAL AGENTS AND CHEMOTHERAPY, Band 36, Nr. 8, August 1992, Seiten 1630-1634; A. KUHLENCORD et al.: "Hexadecylphosphocholine: oral treatment of visceral leishmaniasis in mice"
- BRITISH MEDICAL JOURNAL, Band 291, 14. September 1985, Seiten 704-705; J. EL-ON et al.: "Topical treatment of recurrent cutaneous leishmaniasis with ointment containing paromomycin and methylbenzethonium chloride"
- THE AMERICAN JOURNAL OF TROPICAL MEDICINE AND HYGIENE, Band 35, Nr. 6, November 1986, Seiten 1110-1116, The American Society of Tropical Medicine and Hygiene; J. EL-ON et al.: "Leishmania major: antileishmanial activity of methylbenzethonium chloride"

## Beschreibung

Die vorliegende Erfindung betrifft die Verwendung von Arzneimitteln zur oralen oder topischen Verabreichung bei der Behandlung von Protozoenerkrankungen, insbesondere der Leishmaniasis.

Die Leishmaniasis ist eine Bezeichnung für verschiedene Tropenkranheiten, welche durch Flagellaten der Gattung Leishmania hervorgerufen und durch verschiedene blutsaugende Insekten übertragen werden. Die Erscheinungsformen der Leishmaniasis können viszeral (Kala-Azar), mukokutan (amerikanische Leishmaniasis) oder kutan (Aleppobeule oder diffuse Hautleishmaniasis) sein. Die Inkubationszeit beträgt Wochen bis Monate. Insbesondere bei der Kalar-Azar und der amerikanischen Leishmaniasis wird in unbehandelten Fällen eine sehr hohe Mortalitätsrate beobachtet.

Die heute verwendeten therapeutischen Mittel zur Behandlung von Leishmaniasis sind fünfwertige Antimon-Verbindungen (z.B. Natriumstibogluconat) und aromatische Diamidine. Ein Nachteil dieser Medikamente besteht jedoch darin, daß sie starke Nebenwirkungen wie Übelkeit und Erbrechen aufgrund ihrer hohen Toxizität verursachen. Ferner gibt es bereits Leishmania-Stämme, die Antimon-resistent sind.

Croft et al. (Biochem.Pharmacol. 36 (1987), S. 2633-2636) beschreiben Experimente, in denen die Wirksamkeit von Alkylphosphocholinen gegen Leishmania donovani untersucht wird. Es wurde die Wirkung von Alkylphosphocholinen mit der Wirkung des Standardtherapeutikums Natriumstibogluconat (Pentostam) bei subkutaner Verabreichung getestet. Dabei wurde gefunden, daß Alkylphosphocholine, insbesondere C₂₂-Alkylphosphocholine, eine Aktivität gegen Leishmania zeigten. Es wurde aber auch festgestellt, daß die Alkylphosphocholine, insbesondere Hexadexylphosphocholin, eine hohe Toxizität gegenüber den Versuchstieren, insbesondere gegen Makrophagen, bei therapeutisch wirksamen Dosierungen zeigten, so daß sie bei subkutaner Verabreichung keine echte Alternative zur bekannten Therapie mit Natriumstibogluconat zeigen.

EP-A-O 230 575 beschreibt ein Arzneimittel, das Hexadecylphosphocholin und ggf. Alkylglycerine enthält. Dieses Arzneimittel hat eine cytotoxische Wirksamkeit und wird zur Behandlung von Tumoren eingesetzt. Eine Verwendung zur Behandlung von Leishmaniasis wird weder offenbart noch nahegelegt.

Biochemical Pharmacology 36 (1987), 2633-2636 offenbart die Verwendung von Alkylphosphocholinen zur Behandlung von Leishmaniasis. Die Verabreichung der Alkylphosphocholine erfolgt auf subkutanem Weg (vgl. Seite 2633, linke Spalte, rechter Absatz). Es wird festgestellt, daß die Alkylphosphocholine, insbesondere Hexadecylphosphocholin, eine hohe Toxizität gegenüber Versuchstieren bei therapeutisch wirksamen Dosierungen zeigen. Diese hohe Toxizität führt bei einer subkutanen Verabreichung in Mäusen zu unakzeptablen Nebenwirkungen, wie Hautödemen und Nekrosen. Daher stellt die subkutane Verabreichung von Alkylphosphocholinen keine echte Alternative zur bekannten intravenösen Therapie mit Natriumstibogluconat dar.

EP-A-O 108 565 offenbart Alkylphosphocholine und ihre Verwendung als Antiturnormittel, zur Bekämpfung von Pilzerkrankungen oder zur Verwendung für die Bekämpfung von Pflanzenkrankheiten. Weiterhin findet sich die Offenbarung, daß Alkylphosphocholine eine Wirksamkeit gegen Protozoen, z.B. Tetrahymena, besitzen und daher als Protozoenhemmstoffe bei Tests auf Bakterien, z.B. in Abwässern, eingesetzt werden können. Eine pharmazeutische Verwendung zur Bekämpfung von Protozoenerkrankungen, insbesondere der Leishmaniasis, wird weder offenbart noch nahegelegt.

Aufgabe der vorliegenden Erfindung war es somit, ein Arzneimittel gegen Protozoenerkrankungen, insbesondere Leishmaniasis bereitzustellen, bei dem die Nachteile des Standes der Technik, insbesondere hinsichtlich der schweren Nebenwirkungen zumindestens teilweise beseitigt sind.

Die erfindungsgemäße Aufgabe wird gelöst durch die Verwendung einer oder mehrerer Verbindungen der allgemeinen Formel I 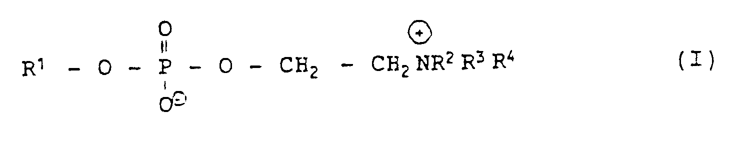 worin R¹ ein gesättigter oder einfach oder mehrfach ungesättigter Kohlenwasserstoffrest mit 12 bis 20 C-Atomen ist und R², R³ und R⁴ unabhängig voneinander Wasserstoff, eine C₁-C₅-Alkylgruppe, eine C₃-C₆-Cycloalkylgruppe oder eine C₁-C₅-Hydroxyalkylgruppe sind, wobei zwei der Reste R², R³ und R⁴ miteinander eine C₂-C₅-Alkylengruppe bilden können, die gegebenenfalls mit einer -O-, -S- oder NR⁵-Gruppe substituiert sein kann, worin R⁵ Wasserstoff, eine C₁-C₅-Alkylgruppe, eine C₃-C₆-Cycloalkylgruppe oder eine C₁-C₅-Hydroxyalkylgruppe ist, sowie gegebenenfalls üblichen pharmazeutischen Hilfs-, Verdünnungs-, Träger- oder/und Füllstoffen zur Herstellung eines Arzneimittels zur oralen oder topischen Verabreichung bei der Behandlung von Leishmaniasis.

In der allgemeinen Formel I kann R¹ verzweigt oder geradkettig sein. Vorzugsweise ist R¹ ein geradkettiger Kohlenwasserstoffrest mit 16 bis 20 C-Atomen, insbesondere ein Hexadecyl-, Octadecyl-, Oleyl-, Elaidyl-, Eicosyl- oder Elcosenyl-cis-(ω-9)rest. Besonders bevorzugt ist R¹ ein Hexadecyl- oder Octadecylrest.

Beispiele für geeignete Reste R², R³ und R⁴ in der Formel I sind etwa Methyl-, Ethyl-, Propyl-, Butyl- und Pentylreste, Cyclopropyl-, Cyclobutyl-, Cyclopentyl- und Cyclohexylreste, Hydroxymethyl-, Hydroxyethyl- und Hydroxyropylreste. Zwei der Reste R², R³ und R⁴ können beispielsweise eine Pyrrolidin-, eine Piperidin- oder eine Moryholingruppe bilden. Vorzugsweise ist mindestens einer der Reste R², R³ und R⁴ von Wasserstoff verschieden, besonders bevorzugt sind alle 3 Reste von Wasserstoff verschieden.

Beispiele für bevorzugte Reste R², R³ und R⁴ sind Methyl-, Ethyl-, Hydroxyethyl- und C₃-C₆-Cycloalkylreste. Wenn einer von R², R³ und R⁴ ein Cycloalkylrest ist, dann sind die anderen beiden Reste vorzugswiese Methylreste. Besonders bevorzugt sind die Reste R², R³ und R⁴ unabhängig voneinander Methyl- oder Ethylreste. Am meisten bevorzugt ist, wenn R², R³ und R⁴ Methylreste sind, so daß eine besonders bevorzugte Verbindungsklasse, die zur Herstellung eines Mittels gegen Protozoenerkrankungen, insbesondere der Leishmaniasis, geeignet ist, Alkylphosphocholine darstellen.

Überraschenderweise wurde festgestellt, daß Verbindungen der allgemeinen Formel I bei oraler oder topischer Applikation keine meßbaren Nebenwirkungen und eine sehr viel höhere Aktivität als intravenös appliziertes Natriumstibogluconat zeigten. Die erfindungsgemäßen Arzneimittel stellen die ersten oralen Therapieformen für Leishmaniasiserkrankungen überhaupt dar und zeigen in der Leber und insbesondere auch in der Milz eine ganz erheblich bessere Wirkung als Pentostam, ein weltweit vervendetes Standardtherapeutikum.

In einer bevorzugten Ausführungsform der vorliegenden Erfindung enthält das orale oder topische Therapeutikum zusätzlich 1 oder mehrere Alkylglycerine der allgemeinen Formel II 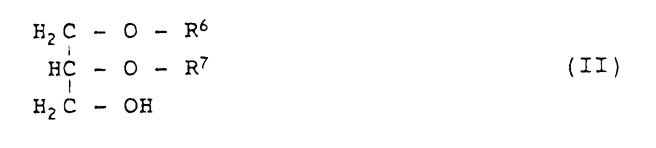 worin einer der Reste R⁶ und R⁷ eine Alkylgruppe mit 2 bis 12 C-Atomen und der andere Rest ein Wasserstoffatom bedeutet. Vorzugsweise wird ein Alkylglyceringemisch verwendet, das Nonyl- bzw. Octylglycerin, Hexyl- bzw. Penylglycerin und Propyl- bzw. Ethylglycerin sowie gegebenenfalls Wasser enthält.

Das erfindungsgemäße Arzneimittel enthält in einer Dosierungseinheit vorzugsweise 5 bis 2000 mg, besonders bevorzugt 10 bis 500 mg von einer oder mehrerer Verbindungen der allgemeinen Formel I. Für die topische Applikation enthält das erfindungsgemäße Arzneimittel vorzugsweise 5 bis 200 mg von einer oder mehreren Verbindungen der allgemeinen Formel I pro ml eines Alkylglycerins der Formel II bzw. eines entsprechenden Alkylglycelingemisches.

Für die orale Applikation wird das erfindungsgemäße Arzneimittel vorzugsweise als Trinklösung mit einer Tagesdosis zwischen 1 und 10 mg/kg von einer oder mehrerer Verbindungen der allgemeinen Formel I formuliert.

Die Herstellung eines oralen Arzneimittels kann erfindungsgemäß andererseits auch erfolgen, indem man eine oder mehrere Verbindungen der allgemeinen Formel I mit üblichen physiologisch verträglichen Füll-, Träger-, Verdünnungs- oder/und Hilfsstoffen bei Temperaturen zwischen 20 und 120 °C vermischt bzw. homogenisiert und gegebenenfalls die so erhaltene Mischung zur Herstellung von Zubereitungen, die pro Dosierungseinheit 10 bis 800 mg von Verbindungen der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt. Der Wirkstoff kann beispielsweise mit einem oder mehreren der folgenden Hilfsstoffe: Stärke, Cellulose, Lactose, Formalin-Kasein, modifizierte Stärke, Magnesiumstearat, Calciumhydrogenphosphat, hochdisperse Kieselsäure, Talkum und Phenoxyethanol vermischt werden. Die erhaltene Mischung kann gegebenenfalls mit einer wäßrigen Lösung, die als Bestandteil beispielsweise Gelatine, Stärke, Polyvinylpyrrolidon, Vinylpyrrolidon-Vinylacetat-Copolymerisat oder/und Polyoxyethylensorbitanmonooleat enthält, granuliert und das Granulat gegebenenfalls mit einem oder mehreren der oben genannten Hilfsstoffe homogenisiert werden. Anschließend kann diese Mischung zu Tabletten verpreßt oder in Kapseln abgefüllt werden, wobei die Kapseln oder Tabletten pro Dosierungseinheit jeweils 10 bis 800 mg Wirkstoff enthalten.

In einer besonders bevorzugten Ausführungsform wird der Wirkstoff mit Sojalecithin sowie gegebenenfalls 0,1 bis 0,5 Gewichtsteilen Phenoxyethanol (bezogen auf 1 Gewichtsteil des Wirkstoffs) bei Temperaturen zwischen 33 bis 37°C in geschmolzenem Hartfett suspendiert und homogenisiert und anschließend wird die Mischung in Hohlzellen ausgegossen, wobei eine Dosierungseinheit 10 bis 800 mg des Wirkstoffs enthält.

Ferner kann man den Wirkstoff bei einer Temperatur zwischen 50 und 120°C, gegebenenfalls in Gegenwart eines oder mehrerer Emulgatoren oder/und 0,1 bis 0,5 Gewichtsteilen Phenoxyethanol (bezogen auf 1 Gewichtsteil des Wirkstoffs) mindestens einem der folgenden Stoffe homogenisieren: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester Gegebenenfalls kann die erhaltene Mischung unter Zusatz eines mehrwertigen niederen (vorzugsweise C₂-C₃) aliphatischen Alkohols (z.B. Ethylenglykol, Diethylenglykol, Propylenglykol, Dipropylenglykol und insbesondere Glycerin) emulgiert werden.

Andererseits kann der Wirkstoff gegebenenfalls in Gegenwart von 0,1 bis 0,5 Gewichtsteilen Phenoxyethanol (bezogen auf 1 Gewichtsteil des Wirkstoffs) sowie gegebenenfalls in Anwesenheit eines Emulgators bei Temperaturen zwischen 30 und 100°C aufgelöst und gegebenenfalls die so erhaltene Lösung mit soviel Wasser oder Pflanzenöl aufgefüllt werden, daß die Endlösung 0,1 bis 5 Gew.-% an Wirkstoff enthält.

Der Wirkstoff kann auch zusammen mit einem Alkylglycerin der allgemeinen Formel II oder einem Gemisch solcher Alkylglycerine sowie gegebenenfalls Wasser vermischt werden, wobei man vorzugsweise 1 bis 30 Gewichtsteile Alkylglycerin der Formel II bzw. eines entsprechenden Alkylgemisches und gegebenenfalls 1 bis 30 Gewichtsteile Wasser, jeweils bezogen auf 1 Gewichtsteil Wirkstoff gemaß allgemeiner Formel I, verwendet.

Die Herstellung von Verbindungen der allgemeinen Formel I ist für Hexadecylphosphocholin in den Beispielen genau beschrieben. Weitere Methoden zur Herstellung von Verbindungen der allgemeinen Formel I sind z.B. in DE-A 27 52 125, DE-A 36 41 379, DE-A 36 41 491, DE-A 40 13 632, DE-A 36 41 377, der darin zitierten Literatur oder in früheren Patentanmeldungen und -schriften der gleichen Anmelderin beschrieben. Auf diese Literatur wird für die vorliegende Patentanmeldung ausdrücklich Bezug genommen.

Die Erfindung soll weiter durch die folgenden Beispiele verdeutlicht werden.

### Beispiel 1

### Herstellung von Hexadexylphosphocholin·H₂O

### a) Hexadecylphosphoethanolamin (Phosphorylierung, Ringschluß und Ringöffnung)

Hexadecanol (1 Mol, 243 g) und Triethylamin (1,8 Mol, 180 g) werden in 1,5 l THF (Tetrahydrofuran) gelöst und tropfenweise zu einer stark gerührten Lösung von Phosphoroxychlorid (1,2 Mol, 184 g) in 120 ml THF so zugegeben, daß die Temperatur im Reaktionsgefäß (Dreihals, 5 l, mit Tropftrichter, Thermometer und Rührer) 10°C nicht übersteigt. Zur Beschleunigung des Vorgangs wird das Reaktionsgefäß mit einer Eis-Kochsalzmischung gekühlt. Unmittelbar nach dem Eintropfen ist die Reaktion abgeschlossen (Nachweis über DSC in Ether: Rf-Werte von 0,8 für das Ausgangsprodukt, von 0,0 für das Reaktionsprodukt nach Hydrolyse mit Wasser).

Man entfernt das Eisbad und tropft in das Reaktionsgemisch unter starkem Rühren eine Lösung von Ethanolamin (1,5 Mol, 92 g) und Triethylamin (1,8 Mol, 180 g) in 1 1 Dioxan so ein, daß die Temperatur im Reaktionsgefäß auf 65 bis 70°C steigt. Dann ist die Ringbildung abgeschlossen (Nachweis durch DSC in Ether: Rf-Wert von 0,2). Man filtriert von ausgefallenem Triethylaminhydrochlorid noch warm ab und versetzt das Filtrat bei 40 bis 50°C mit 1,5 1 2N Ameisensäure. Nach 15 Minuten ist die Ringöffnung abgeschlossen (Nachweis durch DSC in Ether: Rf-Wert 0,0; DSC in Chloroform/Methanol/Essigsäure/Wasser 100:60:20:5 per Vol.: Rf-Wert 0,8). Man kühlt auf -20°C und filtriert vom Niederschlag ab, der aus weitgehend reinem Hexadecylphosphoethanolamin besteht. Bei leichten Verunreinigungen wird eine chromatographische Reinigung angeschlossen. Mikroanalyse (MG 365,50):

| | | | | |
|---|---|---|---|---|
| ber. (%) | C 59,15 | H 11,03 | N 3,83 | P 8,48 |
| gef. (%) | 59,01 | 10,95 | 3,79 | 8,31 |

### Methylierung von Hexadecylphosphoethanolamin

Die nach Beispiel 1 erhaltenen Kristalle werden ohne weitere Reinigung in 1,2 l 2-Propanol und 0,4 l Dichlormethan aufgenommen. Man versetzt die Suspension der Kristalle unter starkem Rühren mit Kaliumcarbonat (4 Mol, 560 g) in 1 l Wasser. Das zweiphasige Reaktionsgemisch wird mit Dimethylsulfat (4 Mol, 500 g) tropfenweise und unter Rühren so versetzt, daß die Temperatur 40°C nicht übersteigt. Die Reaktion ist 60 Minuten nach dem Eintropfen beendet (Nachweis durch DSC in Chloroform/Methanol/25 %igem Ammoniak 50:50:5 per Vol.: Rf-Wert 0,3). Nach Phasenseparation bei 20°C enthält die obere Phase das Produkt. Man entfernt das Lösungsmittel am Rotationsverdampfer unter Vakuum und chromatographiert den viskosen Rückstand an Kieselgel (Merck Art. 7733, Kieselgel 60, Korngröße 0,2 bis 0,5 mm).

### Chromatographie

Kieselgel, 2 kg, werden mit Chloroform/Methanol/25 %igem Ammoniak (200/15/1 per Vol.) versetzt und in eine Chromatographiesäule gefüllt. Man löst das viskose Öl in 800 ml des obigen Lösungsmittelgemisches und gibt das Rohprodukt auf die Säule (unlösliche Anteile werden vorher abfiltriert). Man eluiert mit Fließmitteln steigender Polarität bis die Verunreinigung ausgewaschen sind. Das Produkt wird schließlich mit Chloroform/Methanol/25 %igem Ammoniak (50/50/5 per Vol.) eluiert. Die vereinigten Eluate werden einrotiert und mit Toluol das restliche Wasser entfernt. Der Rückstand wird in 600 ml Dichlormethan aufgenommen und mit 4 l Aceton versetzt. Die bei -20°C abgeschiedenen Kristalle werden mit kaltem Aceton gewaschen, dann mit Pentan und im Vakuum getrocknet. Die Ausbeute an reinem Hexadexylphosphocholin beträgt 250 g (ca. 70 % bezogen auf Hexadexylglycerin).

### Mikroanalyse (MG 407,58):

| | | | | |
|---|---|---|---|---|
| ber. (%): | C 59,27 | H 11,37 | H 3,29 | P 7,28 |
| gef. (%): | 58,98 | 11,31 | 3,21 | 7,11 |

### Herstellung von pharmazeutischen Zubereitungen

### Beispiel für eine Lösung:

25 g 1-n-Propyloxy-2,3-Propandiol, 12,5 g 1-n-Hexyloxy-2,3-propandiol, 12,5 g 1-n-Nonyloxy-2,3-propandiol, 44 g Wasser und 1 g Phenoxyethanol werden gemischt und 5 g Hexadexylphosphocholin in dieser Mischung gelöst. Die Lösung wird durch Filtration über geeignete Filter von sichtbaren Partikeln befreit.
1 g Lösung enthält 50 mg Hexadexylphosphocholin.

### Beispiel für eine Salbe:

5 g Hexadexylphosphocholin werden in 35 g dickflüssigem Paraffin suspendiert, 30 g emulgierender Cetylstearylalkohol und 30 g weiße Vaseline werden zugesetzt und geschmolzen. Diese Schmelze wird bis zum Erkalten gerührt. Eine homogene Wirkstoffverteilung wird durch Bearbeitung der erkalteten Schmelze mittels eines geeigneten Homogenisiergerätes (z.B. Dreiwalzenstuhl) erreicht.
1 g der hydrophilen Salbe enthält 50 mg Hexadexylphosphocholin.

### Beispiel für eine Emulsion:

11,83 g 1--Propyloxy-2,3-propandiol, 5,91 g 1-n-Hexaloxy-2,3-propandiol, 5,91 g 1-n-Nonyloxy-2,3-propandiol, 20,35 g Wasser und 1,0 g Pehnoxyethanol werden gemischt und 5 g Hexadecylphosphocholin in dieser Mischung gelöst. Auf einem Wasserbad werden 30 g weiße Vaseline, 15 g Cetylalkohol und 5 g Sorbitanmonopalmitat geschmolzen, auf 70°C erwärmt und die ebenfalls auf 70°C erwärmte Wirkstofflösung mit Hilfe eines hochtourigen Dispergiergerätes in der Fettphase emulgiert. Anschließend wird unter Rühren die Creme auf 30°C abgekühlt.
1 g Wasser-in-Öl-Creme enthält 50 mg Hexadexylphosphocholin.

### Beispiel für Kapseln:

1,25 kg Hexadecylphosphocholin werden in 5 kg Chloroform gelöst und in dieser Lösung 1,25 kg Aerosil suspendiert. Anschließend wird das Lösungsmittel im Vakuum abgezogen. Die trockene Masse wird durch ein 1 mm-Sieb gegeben und noch einmal im Vakuum bei 30°C getrocknet, um letzte Lösungsmittelrückstände zu entfernen. Dieses Granulat wird in bekannter Weise auf einer geeigneten Kapselmaschine in Gelatine Hartkapseln der Größe 00 zu 500 mg abgefüllt.
Eine Kapsel enthält 250 mg Hexadexylphosphocholin.

### Beispiele für weitere Wirkstoffe

### Beispiel 2

| Octadecylphosphocholin | |
|---|---|
| C₂₃H₅₀NO₄P | MG 435.630 |

### Beispiel 3

| Oleylphosphocholin | |
|---|---|
| C₂₃H₄₈NO₄P | MG 433.614 |

### Beispiel 4

| Elaidylphosphocholin | |
|---|---|
| C₂₃H₄₈NO₄P | MG 433.614 |

### Beispiel 5

| | |
|---|---|
| Hexadecylphospho-(N.N-dimethyl-N-ethyl)ethanolamin | MG 421.603 |

### Beispiel 6

| | |
|---|---|
| Octadecylphospho-(N.N-dimethyl-N-ethyl)ethanolamin | MG 449.657 |

### Beispiel 7

| | |
|---|---|
| Oleylphospho-(N.N-dimethyl-N-ethyl)ethanolamin | MG 447.641 |

### Beispiel 8

| | |
|---|---|
| Elaidylphospho-(N.N-dimethyl-N-ethyl)ethanolamin | MG 447.641 |

### Beispiel 9

| Hexadecylphospho-(N-cyclopropyl-N.N-dimethyl)-ethanolamin | |
|---|---|
| C₂₃H₄₈NO₄P | MG 433.614 |

### Beispiel 10

| Hexadexylphospho-(N-cyclobutyl-N.N-dimethyl)-ethanolamin | |
|---|---|
| C₂₄H₅₀NO₄P | MG 447.641 |

### Beispiel 11

| Hexadecylphospho-(N-cyclopentyl-N.N-dimethyl)-ethanolamin | |
|---|---|
| C₂₅H₅₂NO₄P | MG 461.668 |

### Beispiel 12

| Hexadecylphospho-(N.N-dimethyl-N-hydroxyethyl)-ethanolamin | |
|---|---|
| C₂₂H₄₈NO₅P | MG 437.602 |

### Beispiel 13

| Hexadecylphospho-(N-methyl)-pyrrolidino-ethylester | |
|---|---|
| C₂₃H₄₈NO₄P | MG 433.614 |

### Beispiel 14

| Octadecylphosphoh-(N.N-diethyl-N-methyl)-ethanolamin | |
|---|---|
| C₂₅H₅₄NO₄P | MG 463.684 |

### Beispiel 15

| Octadecylphospho-(N-cyclopropyl-N.N-dimethyl)-ethanolamin | |
|---|---|
| CH₂₅H₅₂NO₄P | MG 461.668 |

### Beispiel 16

| Octadecylphospho-(N-cyclobutyl-N.N-dimethyl)-ethanolamin | |
|---|---|
| C₂₆H₅₄NO₄P | MG 475.695 |

### Beispiel 17

| Octadecylphospho-(N-cyclopentyl-N.N-dimethyl)-ethanolamin | |
|---|---|
| C₂₇H₅₆NO₄P | MG 489.722 |

### Beispiel 18

| Octadecylphospho-(N.N.dimethyl-N-hydroxyethyl)-ethanolamin | |
|---|---|
| C₂₄H₅₂NO₅P | MG 465.656 |

### Beispiel 19

| Octadecylphospho-(N-methyl)-pyrrolidino-ethylester | |
|---|---|
| C₂₅H₅₂NO₄P | MG 461.668 |

### Beispiel 20

| Oleylphospho-(N.N-diethyl-N-methyl)-ethanolamin | |
|---|---|
| C₂₅H₅₂NO₄P | MG 461.668 |

### Beispiel 21

| Oleylphospho-(N-cyclopropyl-N.N-dimethyl)-ethanolamin | |
|---|---|
| C₂₅H₅₀NO₄P | MG 459.652 |

### Beispiel 22

| Oleylphospho-(N-cyclopentyl-N.N-dimethyl)-ethanolamin | |
|---|---|
| C₂₇H₅₄NO₄P | MG 487.706 |

### Beispiel 23

| Oleylphospho-(N.N-dimethyl-N-hydroxyethyl)-ethanolamin | |
|---|---|
| C₂₄H₅₀NO₅P | MG 463.640 |

### Beispiel 24

| Oleylphospho-(N-methyl)-pyrrolidino-ethylester | |
|---|---|
| C₂₅H₅₀NO₄P | MG 459.652 |

### Beispiel 25

| Elaidylphospho-(N-cyclopropyl-N.N-dimethyl)-ethanolamin | |
|---|---|
| C₂₅H₅₀NO₄P | MG 459.652 |

### Beispiel 26

| Elaidylphospho-(N.N-dimethyl-N-hydroxyethyl)-ethanolamin | |
|---|---|
| C₂₄H₅₀NO₅P | MG 463.640 |

### Beispiel 27

| Elaidylphospho-(N-methyl)-pyrrolidino-ethylester | |
|---|---|
| C₂₅H₅₀NO₄P | MG 459.652 |

### Beispiel 28

| Eicosylphosphocholin | |
|---|---|
| C₂₅H₅₄NO₄P | MG 463.684 |

### Beispiel 29

| Eicosylphospho-(N-ethyl-N.N-dimethyl)-ethanolamin | |
|---|---|
| C₂₆H₅₆NO₄P | MG 477.711 |

### Beispiel 30

| Eicosylphospho-(N.N-diethyl-N-methyl)-ethanolamin | |
|---|---|
| C₂₇H₅₄NO₄P | MG 491.738 |

### Beispiel 31

| Eicosylphospho-(N-cyclopropyl-N.N-dimethyl)-ethanolamin | |
|---|---|
| c₂₇H₅₆NO₄P | MG 489.722 |

### Beispiel 32

| Eicosylphospho-(N.N-dimethyl-N-hydroxyethyl)-ethanolamin | |
|---|---|
| C₂₆H5₆NO₅P | MG 493.710 |

### Beispiel 33

| Eicosylphospho-(N.N-dihydroxyethyl-N-methyl)-ethanolamin | |
|---|---|
| C₂₇H₅₈NO₆P | MG 523.736 |

### Beispiel 34

| Eicosylphospho-(N-methyl)-pyrrolidino-ethylester | |
|---|---|
| C₂₇H₅₆NO₄P | MG 489.722 |

### Beispiel 35

| Eicosenyl-cis-(ω-9)-phosphocholin | |
|---|---|
| C₂₅H₅₂NO₄P | MG 461.668 |

### Beispiel 36

| Eicosenyl-cis-(ω-9)-phospho-(N-ethyl-N.N-dimethyl)-ethanolamin | |
|---|---|
| C₂₆H₅₄NO₄P | MG 475.695 |

### Beispiel 37

| Eicosenyl-cis-(ω-9)-phospho-(N-cyclopropyl-N.N-dimethyl)-ethanolamin | |
|---|---|
| C₂₇H₅₄NO₄P | MG 487.706 |

### Beispiel 38

| Eicosenyl-cis-(ω-9)-phospho-(N.N-dimethyl-N-hydroxyethyl)-ethanolamin | |
|---|---|
| C₂₆H₅₄NO₅P | MG 491.694 |

### Beispiel 39

Wirkung verschiedener Leishmaniasis-Medikamente auf das Vorhandensein von Erregern in der Leber von mit L. donovani infizierten Versuchstieren (Ratten)

Es wurde die Wirkung erfindungsgemäßer Phospholipide (Hexadecylphosphocholin, Octadecylphosphocholin und Oleyl-phospho-(N.N-dimethyl-N-ethyl)ethanolamin) mit dem weltweit verwendeten Standardtherapeutikum Pentostam und dem Etherlipid Et₁₈OCH₃ (1-Octadecyl-2-methyl-rac-glycero-3-phosphocholin) verglichen.

Die erfindungsgemäßen Verbindungen und Et1₈OCH₃ wurden oral appliziert, während Pentostam intravenös appliziert wurden. Dabei wurde gefunden, daß die Alkylphosphocholine mit C₁₈- und C₁₆-Kette eine 32-fach höhere Wirksamkeit als das Standardtherapeutikum Pentostam zeigen, während Oleyl-phospho-(N.N-dimethyl-N-ethyl)ethanolamin eine mit Pentostam vergleichbare Wirkung aufweist.

Die Ergebnisse dieses Versuchs sind in Tabelle I gezeigt. Die Anzahl der Erreger pro Leber wurde durch mikroskopische Analyse bestimmt.

**Tabelle I**

| Anzahl der Leishmania-Erreger in der Leber nach einer Therapiezeit von 3 Wochen | | |
|---|---|---|
| | Erreger pro Leber (in Millionen) | relat. Wirksamkeit (Erreger pro Leber nach Pentostamtherapie dividiert durch Erreger/Leber nach Testsubstanzgabe) |
| Kontrolle | 536,9 | --- |
| 1-Octadecyl-2-methyl-rac-glycero-3-phosphocholin¹ | 424,7 | 0,007 |
| Oleyl-phospho-(N.N-dimethyl-N-ethyl)-ethanolamin ¹ | 4,7 | 0,7 |
| Pentostam ² | 3,2 | 1,0 |
| Hexadecylphosphocholin ¹ | 0,1 | 32,0 |
| Octadecylphosphocholin ¹ | 0,1 | 32,0 |

| | | |
|---|---|---|
| ¹ Alkyl-PC und Et₁₈OCH₃ - oral: 5 x 20 mg/kg/Woche für 3 Wochen | | |
| ² Pentostam - i.v.: 5 x 120 mg/kg/Woche für 3 Wochen | | |

In einigen zusätzlichen Experimenten waren nach einmaliger oraler Gabe von 200 mg Hexadecylphosphocholin in der Leber und in der Milz Parasiten mikroskopisch nicht mehr nachweisbar.

### Beispiel 40

### Wirkung verschiedener Leishmaniasis-Medikamente auf das Vorhandensein von Erregern in der Milz

Die Versuchsdurchführung erfolgte wie in Beispiel 39 beschrieben. Die Ergebnisse dieser Versuche sind in Tabelle II gezeigt.

**Tabelle II**

| Anzahl der Leishmania-Erreger in der Milz nach einer Therapiezeit von 3 Wochen | | |
|---|---|---|
| | Erreger pro Milz (in Millionen) | relat. Wirksamkeit (Erreger pro Milz nach Pentostamtherapie dividiert durch Erreger/Milz nach Testsubstanzgabe) |
| Kontrolle | 24,3 | --- |
| 1-Octadecyl-2-methyl-rac-glycero-3-phosphocholin | 6,5 | 0,9 |
| Oleyl-phospho-(N.N-dimethyl-N-ethyl)-ethanolamin | 0,03 | 210,0 |
| Pentostam | 6,3 | 1,0 |
| Hexadecylphosphocholin | 0,01 | 630,0 |
| Octadecylphosphocholin | 0,01 | 630,0 |

Erstaunlich ist die schwache Wirkung von Pentostam in der Milz. Die Alkylphosphocholine sind hier um Faktoren von > 600 wirksamer als die Standardtherapie.

In einigen zusätzlichen Experimenten waren nach einmaliger oraler Gabe von 200 mg Hexadecylphosphocholin Parasiten mikroskopisch nicht mehr nachweisbar.

Tabelle III zeigt den Organspiegel von Hexadecylphosphocholin (C₁₆-O-PC), Octadecylphosphocholin (C₁₈-O-PC) und 1-Octadecyl-2-methyl-rac-glycero-3-phosphocholin (Et₁₈OCH₃) in der Ratte nach oraler Applikation von 50 µmol/Tag für 5 Tage, 2 Tage Pause, dann weitere 4 Tage jeweils 50 µmol/Tag.

**Tabelle III**

| Organ | Menge Substanz (nmol/g Frischgewebe) | | |
|---|---|---|---|
| | C₁₆-O-PC | c₁₈-O-PC | Et₁₈OCH₃ |
| Serum | 130 | 47 | 5 |
| Leber | 272 | 298 | 36 |
| Milz | 410 | 424 | 43 |
| Niere | 853 | 406 | 57 |

Überraschend ist die gute Anreicherung von Hexadecylphosphocholin und von Octadecylphosphocholin in der Milz. Gerade in der Milz hat das Standardtherapeutikum Pentostam eine äußerst geringe Wirksamkeit. Im Vergleich dazu ist Et₁₈OCH₃ in der Milz und in der Leber nur in äußerst geringen Konzentrationen vorhanden.

## Patentansprüche

1. Verwendung von Verbindungen der allgemeinen Formel I 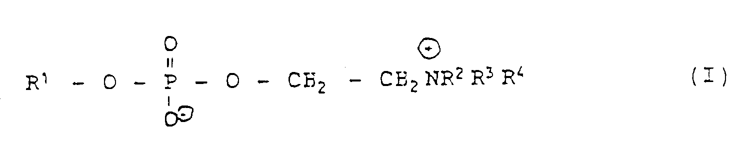 worin R¹ ein gesättigter oder einfach oder mehrfach ungesättigter Kohlenwasserstoffrest mit 12 bis 20 C-Atomen ist und R², R³ und R⁴ unabhängig voneinander Wasserstoff, eine C₁-C₅-Alkylgruppe, eine C₃-C₆-Cycloalkylgruppe oder eine C₁-C₅-Hydroxyalkylgruppe sind, wobei zwei der Reste R², R³ und R⁴ miteinander eine C₂-C₅-Alkylengruppe bilden können, die gegebenenfalls mit einer -O-, -S- oder NR⁵-Gruppe substituiert sein kann, worin R⁵ Wasserstoff, eine C₁-C₅-Alkylgruppe, eine C₃-C₆-Cycloalkylgruppe oder eine C₁-C₅-Hydroxyalkylgruppe ist, sowie gegebenenfalls zusätzlich übliche pharmazeutische Hilfs-, Verdünnungs-, Träger oder/und Füllstoffe enthaltende Gemische davon zur Herstellung eines Arzneimittels für die orale oder topische Behandlung von Leishmaniasis.

2. Verwendung nach Anspruch 1,
**dadurch gekennzeichnet,**
daß R¹ geradkettig ist und 16 bis 20 C-Atome aufweist.

3. Verwendung nach Anspruch 1,
**dadurch gekennzeichnet,**
daß die Reste R², R³ und R⁴ unabhängig voneinander Methyl-, Ethyl-, Hydroxyethyl- oder C₃-C₆-Cycloalkylreste sind.

4. Verwendung nach Anspruch 3,
**dadurch gekennzeichnet,**
daß R², R³ und R⁴ Methylreste sind.

5. Verwendung nach einem der vorhergehenden Ansprüche,
**dadurch gekennzeichnet,**
daß R¹ ein Hexadecyl-, Octadecyl-, Oleoyl-, Elaidyl-, Eicosyl- oder Eicosenyl-cis(ω-9)rest ist.

6. Verwendung nach Anspruch 5,
**dadurch gekennzeichnet,**
daß R¹ ein Hexadecyl- oder Octadecylrest ist.

7. Verwendung nach einem der vorhergehenden Ansprüche,
**dadurch gekennzeichnet,**
daß das Arzneimittel zusätzlich ein oder mehrere Alkylglycerine oder allgemeinen Formel II 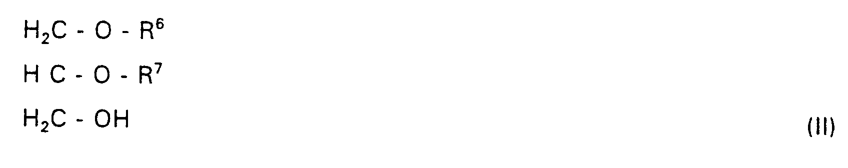 enthält, worin einer der Reste R⁶ und R⁷ eine Alkylgruppe mit 2 bis 12 C-Atomen und der andere Rest ein Wasserstoffatom bedeutet.

8. Verwendung nach Anspruch 7,
**dadurch gekennzeichnet,**
daß das Arzneimittel ein Alkylglycerin-Gemisch aus Nonyl- bzw. Octylglycerin, Hexyl- bzw. Pentylglycerin und Propyl- bzw. Ethylglycerin sowie gegebenenfalls Wasser enthält.

9. Verwendung nach einem der vorhergehenden Ansprüche,
**dadurch gekennzeichnet,**
daß das Arzneimittel in einer Dosierungseinheit 5 bis 2000 mg, insbesondere 10 - 500 mg von einer oder mehreren Verbindungen der allgemeinen Formel I enthält.

10. Verwendung nach einem der vorhergehenden Ansprüche,
**dadurch gekennzeichnet,**
daß es als topisches Arzneimittel 5 - 200 mg von einer oder mehreren Verbindungen der allgemeinen Formel I pro ml eines Alkylglycerins der Formel II bzw. eines entsprechenden Alkylglyceringemisches enthält.

11. Verwendung nach einem der Ansprüche 1 bis 10,
**dadurch gekennzeichnet,**
daß es als Trinklösung mit einer Tagesdosis zwischen 1 und 10 mg/kg von einer oder mehreren Verbindungen der allgemeinen Formel I formuliert wird.

12. Verwendung nach einem der Ansprüche 1 bis 10,
**dadurch gekennzeichnet,**
daß man eine oder mehrere Verbindungen der allgemeinen Formel I mit üblichen physiologisch verträglichen Füll-, Träger-, Verdünnungs- oder/und Hilfsstoffen bei Temperaturen zwischen 20 und 120 °C vermischt bzw. homogenisiert und gegebenenfalls die so erhaltene Mischung zur Herstellung von Zubereitungen, die in einer Dosierungseinheit 10 bis 800 mg von Verbindungen der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

13. Verwendung nach Anspruch 12,
**dadurch gekennzeichnet,**
daß man eine oder mehrere der Verbindungen der allgemeinen Formel I mit einem oder mehreren der folgenden Hilfsstoffe: Stärke, Cellulose, Lactose, Formalin-Casein, modifizierte Stärke, Magnesiumstearat, Calciumhydrogenphosphat, hochdisperse Kieselsäure, Talkum und Phenoxyethanol vermischt, die erhaltene Mischung, gegebenenfalls mit einer wäßrigen Lösung, die als Bestandteil Gelatine, Stärke, Polyvinylpyrrolidon, Vinylpyrrolidon-Vinylacetat-Copolymerisat oder/und Polyoxyethylensorbitanmonooleat enthält, granuliert, das Granulat gegebenenfalls mit einem oder mehreren der oben genannten Hilfsstoffe homogenisiert und diese Mischung zu Tabletten verpreßt oder in Kapseln abfüllt, wobei die Tabletten oder Kapseln in einer Dosierungseinheit jeweils 10 bis 800 mg von Verbindungen der allgemeinen Formel I enthalten.

14. Verwendung nach einem der Ansprüche 12 oder 13,
**dadurch gekennzeichnet,**
daß man eine oder mehrere Verbindungen der allgemeinen Formel I mit Sojalecithin sowie gegebenenfalls 0,1 bis 0,5 Gewichtsteilen Phenoxyethanol (bezogen auf einen Gewichtsteil von Verbindungen der Formel I) bei Temperaturen zwischen 33 - 37 °C in geschmolzenem Harzfett suspendiert und homogenisiert und anschließend die Mischung in Hohlzellen ausgießt, wobei die Dosierungseinheit 10 bis 800 mg von Verbindungen der Formel I enthält.

15. Verwendung nach einem der Ansprüche 12 oder 13,
**dadurch gekennzeichnet,**
daß man eine oder mehrere Verbindungen der allgemeinen Formel I bei einer Temperatur zwischen 50 bis 120 °C, gegebenenfalls in Gegenwart eines oder mehrerer Emulgatoren oder/und 0,1 - 0,5 Gewichtsteilen Phenoxyethanol (bezogen auf 1 Gewichtsteil von Verbindungen der Formel I) mit mindestens einem der folgenden Stoffe homogenisiert: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester, und die homogenisierte Mischung gegebenenfalls unter Zusatz eines mehrwertigen niederen aliphatischen Alkohols emulgiert.

16. Verwendung nach einem der Ansprüche 12 bis 15,
**dadurch gekennzeichnet,**
daß man eine oder mehrere Verbindungen der allgemeinen Formel I gegebenenfalls in Gegenwart von 0,1 - 0,5 Gewichtsteilen Phenoxyethanol (bezogen auf einen Gewichtsteil von Verbindungen der Formel I) sowie gegebenenfalls in Anwesenheit eines Emulgators, bei Temperaturen zwischen 30 - 100 °C auflöst, und gegebenenfalls die so erhaltene Lösung mit soviel Wasser oder Pflanzenöl auffüllt, daß die Endlösung 0,1 - 5 Gewichtsprozent an Verbindungen der Formel I enthält.

17. Verwendung nach einem der Ansprüche 7 bis 16,
**dadurch gekennzeichnet,**
daß man bei der Herstellung zusätzlich noch ein Alkylglycerin der Formel II 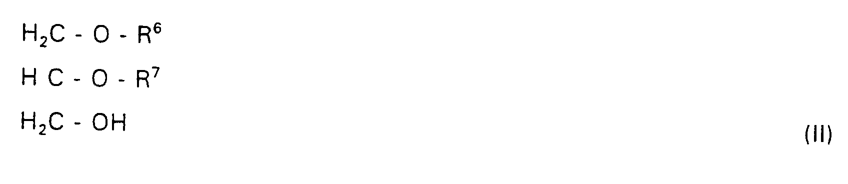 in der einer der Reste R⁶ und R⁷ eine Alkylgruppe mit 2 bis 12 C-Atomen und der andere Rest ein H-Atom bedeutet, oder ein Gemisch solcher Alkylglycerine sowie gegebenenfalls Wasser verwendet, wobei man 1 - 30 Gewichtsteile Alkylglycerin der Formel II bzw. eines entsprechenden Alkylglyceringemisches und gegebenenfalls 1 - 30 Gewichtsteile Wasser, jeweils bezogen auf einen Gewichtsteil an Verbindungen der Formel I, verwendet.

## Claims

1. Use of compounds having the general formula I 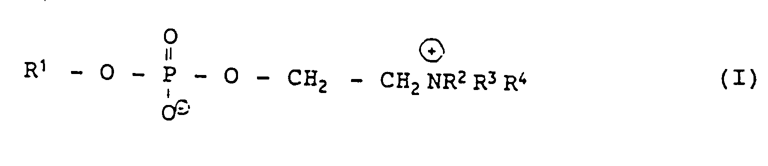 in which R¹ is a saturated or monounsaturated or polyunsaturated hydrocarbon residue with 12 to 20 C atoms and R², R³ and R⁴ denote independently of one another hydrogen, a C₁-C₅ alkyl group, a C₃-C₆ cycloalkyl group or a C₁-C₅ hydroxyalkyl group whereby two of the residues R², R³ and R⁴ can together form a C₂-C₅ alkylene group which can optionally be substituted with an -O-, -S- or NR⁵ group, in which R⁵ is hydrogen, a C₁-C₅ alkyl group, a C₃-C₆ cycloalkyl group or a C₁-C₅ hydroxyalkyl group as well as optionally mixtures thereof containing additional common pharmaceutical auxiliary, diluting, carrier or/and filling substances for the production of a pharmaceutical preparation for the oral or topical treatment of leishmaniasis.

2. Use as claimed in claim 1,
**wherein**
R¹ is straight-chained and has 16 to 20 C atoms.

3. Use as claimed in claim 1,
**wherein**
the residues R², R³ and R⁴ are independently of each other methyl, ethyl, hydroxyethyl or C₃-C₆ cycloalkyl residues.

4. Use as claimed in claim 3,
**wherein**
R², R³ and R⁴ are methyl residues.

5. Use as claimed in one of the previous claims,
**wherein**
R¹ is a hexadecyl, octadecyl, oleoyl, elaidyl, eicosyl or eicosenyl-cis(ω-9) residue.

6. Use as claimed in claim 5,
**wherein**
R¹ is a hexadecyl or octadecyl residue.

7. Use as claimed in one of the previous claims,
**wherein**
the pharmaceutical preparation additionally contains one or several alkyl glycerols having the general formula II 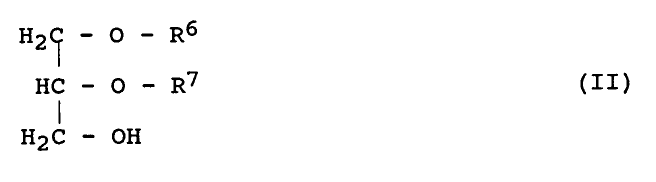 in which one of the residues R⁶ and R⁷ denotes an alkyl group with 2 to 12 C atoms and the other residue denotes a hydrogen atom.

8. Use as claimed in claim 7,
**wherein**
the pharmaceutical preparation contains an alkyl glycerol mixture of nonyl or octyl glycerol, hexyl or pentyl glycerol and propyl or ethyl glycerol as well as, if desired, water.

9. Use as claimed in one of the previous claims,
**wherein**
the pharmaceutical preparation contains 5 to 2000 mg, in particular 10 to 500 mg, of one or several compounds having the general formula I in one dosage unit.

10. Use as claimed in one of the previous claims,
**wherein**
it contains as a topical pharmaceutical preparation 5 - 200 mg of one or several compounds of the general formula I per ml of an alkyl glycerol having the formula II or of a corresponding alkyl glycerol mixture.

11. Use as claimed in one of the claims 1 to 10,
**wherein**
it is formulated as a drinking solution having a daily dose between 1 and 10 mg/kg of one or several compounds of the general formula I.

12. Use as claimed in one of the claims 1 to 10,
**wherein**
one or several compounds of the general formula I are mixed or homogenized with common physiologically tolerated filling, carrier, dilution or/and auxiliary substances at temperatures between 20 and 120°C and optionally in order to prepare preparations which contain 10 to 800 mg of compounds of formula I in one dosage unit, the mixture thus obtained is poured into hollow cells of an appropriate size or filled into capsules of an appropriate size or granulated and then optionally pressed into tablets with addition of further common auxiliary substances.

13. Use as claimed in claim 12,
**wherein**
one or several compounds of the general formula I are mixed with one or several of the following auxiliary substances: starch, cellulose, lactose, formalin-casein, modified starch, magnesium stearate, calcium hydrogenphosphate, highly-dispersed silicic acid, talcum and phenoxyethanol, the mixture obtained is optionally granulated with an aqueous solution containing the constituents gelatin, starch, polyvinyl pyrrolidone, vinyl-pyrrolidone-vinyl acetate copolymer or/and polyoxyethylene sobitan monooleate, the granulate is homogenized, if desired, with one or several of the aforementioned auxiliary substances and this mixture is pressed into tablets or filled into capsules whereby the tablets or capsules each contain 10 to 800 mg of the compounds of the general formula I in one dosage unit.

14. Use as claimed in one of the claims 12 or 13,
**wherein**
one or several compounds of the general formula I are suspended with soybean lecithin as well as optionally with 0.1 to 0.5 parts by weight phenoxyethanol (in relation to one part by weight of compounds of the general formula I) at temperatures between 33 - 37°C in melted resin fat and homogenized and subsequently the mixture is poured into hollow cells whereby the dosage unit contains 10 to 800 mg of compounds of formula I.

15. Use as claimed in one of the claims 12 or 13,
**wherein**,
one or several compounds of the general formula I are homogenized at a temperature between 50 and 120°C, optionally in the presence of one or several emulsifiers or/and 0.1 - 0.5 parts by weight phenoxyethanol (in relation to 1 part by weight of compounds of formula I) with at least one of the following substances: paraffin, vaseline, aliphatic alcohol with 12 to 25 C atoms, sorbitan monopalmitate, aliphatic monocarboxylic acid with 15 to 20 C atoms, polyoxyethylene polyol fatty acid ester and the homogenized mixture is emulsified optionally with addition of a multivalent lower aliphatic alcohol.

16. Use as claimed in one of the claims 12 to 15,
**wherein**
one or several compounds of the general formula I are dissolved at temperatures between 30 - 100°C, optionally in the presence of 0.1 - 0.5 parts by weight phenoxyethanol (in relation to one part by weight of compounds of formula I) as well as optionally in the presence of an emulsifier and if desired, the solution thus obtained is filled up with sufficient water or vegetable oil so that the final solution contains 0.1 - 5 percent by weight of compounds of formula I.

17. Use as claimed in one of the claims 7 to 16,
**wherein**
an alkyl glycerol is additionally used in the production having the formula II 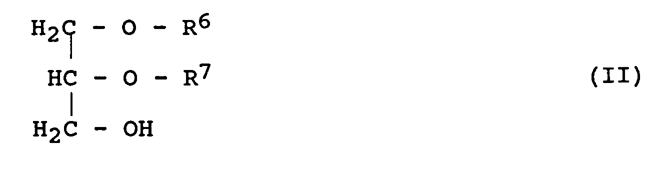 in which one of the residues R⁶ and R⁷ denotes an alkyl group with 2 to 12 C atoms and the other residue denotes a hydrogen atom, or a mixture of such alkyl glycerols and optionally water is used whereby 1 - 30 parts by weight of an alkyl glycerol of formula II or of a corresponding alkyl glycerol mixture and optionally 1 - 30 parts by weight of water, each in relation to 1 part by weight of compounds of formula I, are used.

## Revendications

1. Utilisation de composés de formule générale I 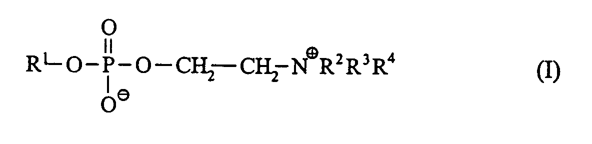 dans laquelle R¹ est un reste hydrocarboné saturé, monoinsaturé ou polyinsaturé de 12 à 20 atomes de carbone et R³ et R⁴ sont indépendamment les uns des autres un atome d'hydrogène, un groupe alkyle en C₁-C₅, un groupe cycloalkyle en C₃-C₆ ou un groupe hydroxyalkyle en C₁-C₅, deux des restes R², R³ et R⁴ pouvant former ensemble un groupe alkylène en C₂-C₅ pouvant éventuellement être substitué par un groupe -O-, -S- ou -NR⁵-, où R⁵ est un atome d'hydrogène, un groupe alkyle en C₁-C₅, un groupe cycloalkyle en C₃-C₆ ou un groupe hydroxyalkyle en C₁-C₅, et de leurs mélanges contenant éventuellement en outre des agents auxiliaires, des diluants, des supports et/ou des charges pharmaceutiques classiques, pour la préparation d'un médicament destiné au traitement par voie orale ou topique de la leishmaniose.

2. Utilisation selon la revendication 1, caractérisée en ce que R¹ est linéaire et contient 16 à 20 atomes de carbone.

3. Utilisation selon la revendication 1, caractérisée en ce que les restes R², R³ et R⁴ sont indépendamment les uns des autres des restes méthyle, éthyle, hydroxyéthyle ou cycloalkyle en C₃-C₆.

4. Utilisation selon la revendication 3, caractérisée en ce que R³ et R⁴ sont des restes méthyle.

5. Utilisation selon l'une des revendications précédentes, caractérisée en ce que R¹ est un reste hexadécyle, octadécyle, oléoyle, élaïdyle, eicosyle ou cis(ω-9)-eicosényle.

6. Utilisation selon la revendication 5; caractérisée en ce que R¹ est un reste hexadécyle ou octadécyle.

7. Utilisation selon l'une des revendications précédentes, caractérisée en ce que le médicament contient en outre un ou plusieurs alkylglycérols de formule générale II 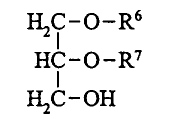 dans laquelle l'un des restes R⁶ et R⁷ représente un groupe alkyle de 2 à 12 atomes de carbone et l'autre représente un atome d'hydrogène.

8. Utilisation selon la revendication 7, caractérisée en ce que le médicament contient un mélange d'alkylglycérols constitué de nonyl- ou octylglycérol, d'hexyl- ou pentylglycérol et de propyl- ou éthylglycérol et éventuellement de l'eau.

9. Utilisation selon l'une des revendications précédentes, caractérisée en ce que le médicament contient dans une unité de prise 5 à 2000 mg, en particulier 10 à 500 mg d'un ou plusieurs composés de formule générale I.

10. Utilisation selon l'une des revendications précédentes, caractérisée en ce que le médicament utilisé par voie topique contient 5 à 500 mg d'un ou plusieurs composés de formule générale I par ml d'un alkylglycérol de formule II ou d'un mélange correspondant d'alkylglycérols.

11. Utilisation selon l'une des revendications 1 à 10, caractérisée en ce que l'on formule une solution à boire avec une dose journalière comprise entre 1 et 10 mg/kg d'un ou plusieurs composés de formule générale I.

12. Utilisation selon l'une des revendications 1 à 10, caractérisée en ce que l'on mélange ou homogénéise un ou plusieurs composés de formule générale I avec des charges, des supports, des diluants et/ou des agents auxiliaires physiologiquement acceptables classiques à des températures comprises entre 20 et 120°C et éventuellement, pour préparer des compositions contenant 10 à 800 mg de composés de formule I par unité de prise, on verse le mélange ainsi obtenu dans des cellules creuses de taille appropriée, ou on en remplit des capsules de taille appropriée, ou on le granule, puis on le presse en comprimés, éventuellement après avoir ajouté d'autres agents auxiliaires classiques.

13. Utilisation selon la revendication 12, caractérisée en ce que l'on mélange un ou plusieurs composés de formule générale I avec un ou plusieurs des agents auxiliaires suivants: de l'amidon, de la cellulose, du lactose, de la caséineformol, de l'amidon modifié, du stéarate de magnésium, de l'hydrogénophosphate de calcium, de l'acide silicique très dispersé, du talc et du phénoxyéthanol, on granule le mélange obtenu, éventuellement avec une solution aqueuse contenant comme constituants de la gélatine, de l'amidon, de la polyvinylpyrrolidone, un copolymère vinylpyrrolidone-acétate de vinyle et/ou du monooléate de polyoxyéthylènesorbitane, on homogénéise le produit granulé éventuellement avec un ou plusieurs des agents auxiliaires précités et on presse ce mélange en comprimés ou on en remplit des capsules, les comprimés ou les capsules contenant par unité de prise 10 à 800 mg de composés de formule générale I.

14. Utilisation selon l'une des revendications 12 ou 13, caractérisée en ce que l'on met en suspension et on homogénéise un ou plusieurs composés de formule générale I avec de la lécithine de soja et éventuellement 0,1 à 0,5 partie en masse de phénoxyéthanol (pour 1 partie en masse de composés de formule I) à des températures comprises entre 33 et 37°C dans de la graisse de résine fondue, puis on verse le mélange dans des cellules creuses, l'unité de prise contenant 10 à 800 mg de composés de formule I.

15. Utilisation selon l'une des revendications 12 ou 13, caractérisée en ce que l'on homogénéise un ou plusieurs composés de formule générale I, à une température comprise entre 50 et 120°C, éventuellement en présence d'un ou plusieurs émulsionnants et/ou de 0,1 à 0,5 partie en masse de phénoxyéthanol (pour 1 partie en masse de composés de formule I) avec au moins l'une des substances suivantes: de la paraffine, de la vaseline, un alcool aliphatique de 12 à 25 atomes de carbone, du monopalmitate de sorbitane, un acide monocarboxylique aliphatique de 15 à 20 atomes de carbone, un ester d'acide gras de polyoxyéthylènepolyol, et on émulsionne le mélange homogénéisé en ajoutant éventuellement un polyalcool aliphatique inférieur.

16. Utilisation selon l'une des revendications 12 à 15, caractérisée en ce que l'on dissout un ou plusieurs composés de formule générale I, éventuellement en présence de 0,1 à 0,5 partie en masse de phénoxyéthanol (pour une partie en masse de composés de formule I) et éventuellement en présence d'un émulsionnant, à des températures comprises entre 30 et 100°C, et on complète la solution ainsi obtenue avec une quantité d'eau ou d'huile végétale telle que la solution finale contient 0,1 à 5 % en masse de composés de formule I.

17. Utilisation selon l'une des revendications 7 à 16, caractérisée en ce que, lors de la préparation, on utilise en outre un alkylglycérol de formule générale II 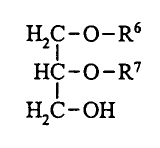 dans laquelle l'un des restes R⁶ et R⁷ représente un groupe alkyle de 2 à 12 atomes de carbone et l'autre représente un atome d'hydrogène, ou un mélange de tels alkylglycérols et éventuellement de l'eau, en utilisant 1 à 30 parties en masse d'alkylglycérol de formule II ou d'un mélange d'alkylglycérols correspondants et éventuellement 1 à 30 parties en masse d'eau, toujours pour 1 partie en masse de composés de formule I.
